(19) Europäisches Patentamt
European Patent Office
Office européen des brevets

(11) EP 4 393 903 B1

# (12) EUROPEAN PATENT SPECIFICATION

(45) Date of publication and mention of the grant of the patent:
**29.10.2025 Bulletin 2025/44**

(21) Application number: **22217073.0**

(22) Date of filing: **29.12.2022**

(51) International Patent Classification (IPC):
***C07C 5/25*** *(2006.01)* ***B01J 21/04*** *(2006.01)*
***C07C 11/107*** *(2006.01)* ***C07C 7/148*** *(2006.01)*
***C07C 11/113*** *(2006.01)* ***B01J 23/92*** *(2006.01)*
***B01J 38/02*** *(2006.01)* ***B01J 38/04*** *(2006.01)*

(52) Cooperative Patent Classification (CPC):
(C-Sets available)
**C07C 5/2512; B01J 21/04; B01J 23/92; B01J 38/02; B01J 38/04; C07C 7/148;**
C07C 2521/04 (Cont.)

## (54) METHOD FOR PURIFYING LINEAR ALPHA OLEFINS

VERFAHREN ZUR REINIGUNG VON LINEAREN ALPHA-OLEFINEN

PROCÉDÉ DE PURIFICATION D'ALPHA-OLÉFINES LINÉAIRES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**03.07.2024 Bulletin 2024/27**

(73) Proprietor: **SABIC Global Technologies B.V.**
**4612 PX Bergen op Zoom (NL)**

(72) Inventors:
- **Viswanath, Vinu**
  **562125 Bengaluru (IN)**
- **Chakraborty, Debashis**
  **562125 Bengaluru (IN)**
- **Ghosh, Ashim Kumar**
  **562125 Bangalore (IN)**
- **Bawareth, Bander**
  **562125 Bangalore (IN)**
- **Banke, Jonathan**
  **562125 Bengaluru (IN)**
- **Al-Thobaity, Abdullah**
  **562125 Bengaluru (IN)**
- **Sadeq, Raeid**
  **562125 Bengaluru (IN)**

(74) Representative: **Hoeger, Stellrecht & Partner Patentanwälte mbB**
**Uhlandstrasse 14c**
**70182 Stuttgart (DE)**

(56) References cited:
**DE-A1- 10 336 015 RU-C1- 2 206 557**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 5/2512, C07C 11/113;**
**C07C 7/148, C07C 11/107**

## Description

TECHNOLOGICAL FIELD

The present disclosure relates to methods for purifying a linear alpha olefin product stream from an oligomerization reaction.

BACKGROUND

Linear olefins are a class of hydrocarbons useful as raw materials in the petrochemical industry and among these the linear alpha olefins, unbranched olefins whose double bond is located at a terminus of the chain, form an important subclass. Linear alpha olefins can be converted to linear primary alcohols by hydroformylation. Hydroformylation can also be used to prepare aldehydes, which in turn can be oxidized to afford synthetic fatty acids, especially those with an odd carbon number, useful in the production of lubricants. Linear alpha olefins are also used in the production of detergents, such as linear alkylbenzenesulfonates, which are prepared by Fiedel-Crafts reaction of benzene with linear olefins followed by sulfonation. Another important use of linear alpha olefins relates to production of linear low-density polyethylene (LLDPE) through catalytic co-polymerization with ethylene.

Preparation of alpha olefins is based largely on oligomerization of ethylene, which has a corollary that the alpha-olefins produced have an even number of carbon atoms. Oligomerization processes for ethylene mainly utilize organoaluminum compounds or transition metals as catalysts. Oligomerization methods are typically carried out in the presence of a catalyst that includes a zirconium component, such as zirconium tetraisobutyrate, and an aluminum component as activator, such as ethyl aluminum sesquichloride. Typically, the effluent from the reactor used to produce the linear alpha olefins is directed to one or more distillation columns to separate the various fractions of linear alpha olefins.

It is desirable to purify the alpha olefin products of oligomerization reactions to a very high purity level, such as a purity level of greater than 99.5 wt.%. Achieving such a high degree of purity can be challenging using conventional separation processes due to the presence of impurities with a boiling point very close to the boiling point of the target alpha olefin products. Documents RU 2 206 557 and DE 10 336 015 disclose the purification of 1-hexene/2-ethyl-1-butene by the selective isomerization of the 2-ethyl-1-butene. There remains a need in the art for improvements in separation processes for such products.

BRIEF SUMMARY

Example implementations of the present disclosure are directed to processes for purifying a linear alpha olefin product stream that involve use of a modified alumina to isomerize 2-ethyl-1-butene to an isomer that is more easily removed from 1-hexene by distillation. In this manner, use of the modified alumina catalyst can improve separation efficiency of the distillation column and enable purification of the target linear alpha olefin to very high purity levels. The present disclosure also provides a catalytic process for selective isomerization of 2-ethyl-1-butene in a linear alpha olefin product stream that includes periodically regenerating the modified alumina catalyst using a non-oxidizing environment for a time period and at a temperature that enables continued use of the catalyst at peak performance levels.

The present disclosure includes, without limitation, the following embodiments.

Embodiment 1: A catalytic process for selective isomerization of 2-ethyl-1-butene in a linear alpha olefin product stream, comprising: feeding a linear alpha olefin product stream comprising at least one linear alpha olefin, such as 1-hexene, and 2-ethyl-1-butene to a reactor housing a modified alumina catalyst to isomerize at least a portion of the 2-ethyl-1-butene to cis- or trans-3-methyl-2-pentene, the period during which said feeding occurs being time on stream; withdrawing an effluent from the reactor containing less 2-ethyl-1-butene than the linear alpha olefin product stream; and periodically regenerating the modified alumina catalyst by stopping the feeding step and introducing a non-oxidizing environment to the reactor at time on stream intervals of no more than about 200 hours, wherein the reactor is subjected to the non-oxidizing environment for a time period of about 15 hours or greater and wherein the non-oxidizing environment has a temperature of about 250 °C or greater for at least a portion of the time period.

Embodiment 2: The catalytic process of Embodiment 1, wherein the non-oxidizing environment comprises a vacuum environment or an inert gas environment, such as nitrogen or a noble gas environment.

Embodiment 3: The catalytic process of Embodiment 1 or 2, wherein the regenerating comprises passing an inert gas through the reactor in two sequential stages comprising i) a first lower temperature stage comprising passing the inert gas through the reactor at an inert gas temperature of about 200 °C or less for a time period of about 2 hours or greater; and ii) a second higher temperature stage comprising passing the inert gas through the reactor at an inert gas temperature of about 250 °C or greater for a time period of about 8 hours or greater.

Embodiment 4: The catalytic process of any one of Embodiments 1 to 3, wherein the first lower temperature stage comprises one or more of: passing the inert gas through the reactor at an inert gas temperature of about 150 °C or less;

passing the inert gas through the reactor at an inert gas temperature of about 40 °C to about 150 °C; passing the inert gas through the reactor at multiple inert gas temperatures ranging from about 40 °C to about 150 °C, wherein the inert gas temperature is increased to each of the multiple inert gas temperatures at a rate of about 1 to about 5 °C/min; passing the inert gas through the reactor for a time period of about 4 hours or greater; and passing the inert gas through the reactor for a time period of about 2 to about 6 hours.

Embodiment 5: The catalytic process of any one of Embodiments 1 to 4, wherein the second higher temperature stage comprises one or more of: passing the inert gas through the reactor at an inert gas temperature of about 300 °C or less; passing the inert gas through the reactor at an inert gas temperature of about 250 °C to about 300 °C; passing the inert gas through the reactor for a time period of about 14 hours or less; and passing the inert gas through the reactor for a time period of about 8 to about 12 hours.

Embodiment 6: The catalytic process of any one of Embodiments 1 to 5, wherein the transition from the first lower temperature stage to the second higher temperature stage occurs at a rate of about 1 to about 5 °C/min.

Embodiment 7: The catalytic process of any one of Embodiments 1 to 6, wherein the inert gas flow rate is about 120 to about 300 $cm^3$/min.

Embodiment 8: The catalytic process of any one of Embodiments 1 to 7, further comprising periodically regenerating the modified alumina catalyst by stopping the feeding step and introducing an oxidizing gas to the reactor.

Embodiment 9: The catalytic process of any one of Embodiments 1 to 8, wherein the oxidizing gas comprises oxygen at a gas temperature of about 300 °C to about 450 °C, optionally introducing the oxidizing gas for a time period of 1 to 24 hours.

Embodiment 10: The catalytic process of any one of Embodiments 1 to 9, wherein the modified alumina catalyst is capable of converting about 80 wt.% or greater of 2-ethyl-1-butene to cis- or trans-3-methyl-2-pentene at a pressure of about 0.1 - 10 barg, a temperature of about 40 - 100 °C, and a Liquid Hourly Space Velocity of about 0.5 - 10 $h^{-1}$, and wherein about 3 wt.% or less of 1-hexene is converted to a different isomer under the same reaction conditions.

Embodiment 11: The catalytic process of any one of Embodiments 1 to 10, further comprising feeding the effluent from the reactor to a distillation column to produce an overhead stream comprising the linear alpha olefin and a bottoms stream comprising cis- or trans-3-methyl-2-pentene.

Embodiment 12: The catalytic process of any one of Embodiments 1 to 11, wherein the overhead stream comprises about 99.5 wt.% 1-hexene or greater and about 0.15 wt.% of 2-ethyl-1-butene or an isomer thereof or less.

Embodiment 13: The catalytic process of any one of Embodiments 1 to 12, wherein the modified alumina catalyst has about 0.01 to about 0.5 mmol/g catalyst of Bronsted acid sites as determined by FTIR spectroscopy using pyridine as probe molecule.

Embodiment 14: The catalytic process of any one of Embodiments 1 to 13, wherein the modified alumina catalyst has about 0.04 to about 0.2 mmol/g catalyst of Bronsted acid sites as determined by FTIR spectroscopy using pyridine as probe molecule.

Embodiment 15: A catalytic process for selective isomerization of 2-ethyl-1-butene in a linear alpha olefin product stream, comprising: feeding a linear alpha olefin product stream comprising at least one linear alpha olefin, such as 1-hexene, and 2-ethyl-1-butene to a reactor housing a modified alumina catalyst to isomerize at least a portion of the 2-ethyl-1-butene to cis- or trans-3-methyl-2-pentene; and withdrawing an effluent from the reactor containing less 2-ethyl-1-butene than the linear alpha olefin product stream, wherein the modified alumina catalyst has about 0.01 to about 0.5 mmol/g catalyst of Brønsted acid sites as determined by FTIR spectroscopy using pyridine as probe molecule, such as about 0.04 to about 0.2 mmol/g catalyst of Brønsted acid sites as determined by FTIR spectroscopy using pyridine as probe molecule.

Embodiment 16: A catalytic reactor system for selective isomerization of 2-ethyl-1-butene in a linear alpha olefin product stream, comprising a catalytic reactor containing a bed of a modified alumina catalyst, wherein the modified alumina catalyst has about 0.01 to about 0.5 mmol/g catalyst of Brønsted acid sites as determined by FTIR spectroscopy using pyridine as probe molecule, such as about 0.04 to about 0.2 mmol/g catalyst of Brønsted acid sites as determined by FTIR spectroscopy using pyridine as probe molecule; the catalytic reactor in fluid communication with a source of alpha olefin product comprising at least one linear alpha olefin, such as 1-hexene, and 2-ethyl-1-butene. Optionally, the source of alpha olefin product is the effluent from an oligomerization reactor.

These and other features, aspects, and advantages of the present disclosure will be apparent from a reading of the following detailed description together with the accompanying figures, which are briefly described below. The present disclosure includes any combination of two, three, four or more features or elements set forth in this disclosure, regardless of whether such features or elements are expressly combined or otherwise recited in a specific example implementation described herein. This disclosure is intended to be read holistically such that any separable features or elements of the disclosure, in any of its aspects and example implementations, should be viewed as combinable, unless the context of the disclosure clearly dictates otherwise.

It will therefore be appreciated that this Brief Summary is provided merely for purposes of summarizing some example implementations so as to provide a basic understanding of some aspects of the disclosure. Accordingly, it will be

appreciated that the above described example implementations are merely examples and should not be construed to narrow the scope or spirit of the disclosure in any way. Other example implementations, aspects and advantages will become apparent from the following detailed description taken in conjunction with the accompanying figures which illustrate, by way of example, the principles of some described example implementations.

## BRIEF DESCRIPTION OF THE FIGURES

Having thus described aspects of the disclosure in the foregoing general terms, reference will now be made to the accompanying figures, which are not necessarily drawn to scale, and wherein:

FIG. 1 is a block diagram of an ethylene oligomerization system according to an example implementation of the present disclosure;
FIG. 2 is block diagram of an example of a purification process of 1 -hexene according to an example implementation of the present disclosure;
FIG. 3 graphically illustrates 1-hexene and 2E1B conversion rates over time during the experiment of Example 1;
FIG. 4 graphically illustrates 1-hexene and 2E1B conversion rates over time during the experiment of Example 2;
FIG. 5 graphically illustrates 1-hexene and 2E1B conversion rates over time during the experiment of Example 3; and
FIG. 6 graphically illustrates the FTIR spectra for unmodified gamma alumina.

## DETAILED DESCRIPTION

Some implementations of the present disclosure will now be described more fully hereinafter with reference to the accompanying figures, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art. Like reference numerals refer to like elements throughout.

Unless specified otherwise or clear from context, references to first, second or the like should not be construed to imply a particular order. A feature described as being above another feature (unless specified otherwise or clear from context) may instead be below, and vice versa; and similarly, features described as being to the left of another feature else may instead be to the right, and vice versa. Also, while reference may be made herein to quantitative measures, values, geometric relationships or the like, unless otherwise stated, any one or more if not all of these may be absolute or approximate to account for acceptable variations that may occur, such as those due to engineering tolerances or the like.

All ranges disclosed herein are inclusive of the endpoints, and the endpoints are independently combinable with each other (e.g., ranges of "up to 25 wt. %, or, more specifically, 5 wt. % to 20 wt. %", is inclusive of the endpoints and all intermediate values of the ranges of "5 wt. % to 25 wt. %," etc.). "Combination" is inclusive of blends, mixtures, alloys, reaction products, and the like.

The terms "about" or "approximately" are defined as being close to as understood by one of ordinary skill in the art. In one non-limiting embodiment, the terms are defined to be within 10%, preferably within 5%, more preferably within 1%, and most preferably within 0.5%.

As used herein, unless specified otherwise or clear from context, the "or" of a set of operands is the "inclusive or" and thereby true if and only if one or more of the operands is true, as opposed to the "exclusive or" which is false when all of the operands are true. Thus, for example, "[A] or [B]" is true if [A] is true, or if [B] is true, or if both [A] and [B] are true. Further, the articles "a" and "an" mean "one or more," unless specified otherwise or clear from context to be directed to a singular form.

The present disclosure provides a modified alumina catalyst having Brønsted acid sites that enable the catalyst to selectively isomerize 2-ethyl-1-butene to cis- or trans-3-methyl-2-pentene in the presence of 1-hexene in order to increase the purity level of 1-hexene produced by ethylene oligomerization. Without being bound by theory of operation, it is believed that the strength and density of Bronsted acid sites in the catalyst play an important role in the selective isomerization. In certain embodiments, the modified alumina catalyst is characterized by having 0.01 to about 0.5 mmol/g catalyst of Bronsted acid sites as determined by FTIR spectroscopy using pyridine as probe molecule.

The present disclosure also provides a catalytic process for selective isomerization of 2-ethyl-1-butene in a linear alpha olefin product stream that includes periodically regenerating the modified alumina catalyst using a non-oxidizing environment for a time period and at a temperature that enables continued use of the catalyst at peak performance levels.

### Ethylene Oligomerization Process and System

Linear alpha olefins (LAOs) are olefins with a chemical formula $C_xH_{2x}$, distinguished from other mono-olefins with

a similar molecular formula by linearity of the hydrocarbon chain and the position of the double bond at the primary or alpha position. Linear alpha olefins comprise a class of industrially important alpha-olefins, including 1-butene, 1-hexene, 1-octene, 1-decene, 1-dodecene, 1-tetradecene, 1-hexadecene, 1-octadecene, and higher blends of $C_{20}$-$C_{24}$, $C_{24}$-$C_{30}$, and $C_{20}$-$C_{30}$ olefins. Linear alpha olefins are useful intermediates for the manufacture of detergents, synthetic lubricants, copolymers, plasticizers, and many other important products.

Existing processes for the production of linear alpha olefins typically rely on the oligomerization of ethylene. For example, linear alpha olefins can be prepared by the catalytic oligomerization of ethylene in the presence of a Ziegler-Natta-type catalyst or non-Ziegler-Natta-type catalyst.

Oligomerization can occur at temperatures of 10 to 200° C, for example, 20 to 100° C, for example, 50 to 90° C, for example, 55 to 80° C, for example, 60 to 70° C. Operating pressures can be 1 to 5 MegaPascals (MPa), for example, 2 to 4 MPa. The process can be continuous and mean residence times can be 10 minutes to 20 hours, for example 30 minutes to 4 hours, for example, 1 to 2 hours. Residence times can be chosen so as to achieve the desired conversion at high selectivity.

The process can be conducted in solution using an inert solvent, which is advantageously non-reactive with the catalyst composition. Examples of desirable organic solvents can include, but are not limited to, aromatic hydrocarbon solvents which can be unsubstituted or substituted with halogens, for example, toluene, benzene, xylene, monochlorobenzene, dichlorobenzene, chlorotoluene; aliphatic paraffin hydrocarbons, for example, pentane, hexane, heptane, octane, nonane, decane; alicyclic hydrocarbon compounds, for example, cyclohexane, decahydronaphthalene; and halogenated alkanes, for example, dichloroethane and dichlorobutane.

The process can be carried out in any reactor, such as a loop reactor, a plug-flow reactor, or a bubble column reactor. Oligomerization of ethylene is an exothermic reaction that can be cooled by a surplus flow of ethylene. The gases leaving at a top portion of the reactor can be cooled using a series of external coolers and condensers. The gas phase, after further cooling, can be recycled.

A bottom stream leaving the oligomerization reactor from a bottom portion can contain the active catalyst and unreacted ethylene. The reaction can be terminated to avoid undesirable side reactions by removing catalyst components from the organic phase through extraction with a caustic aqueous phase. Contact with the caustic aqueous phase can result in formation of nonreactive minerals corresponding to the catalyst components.

The organic phase, after passage through the catalyst removal system, can pass through a molecular sieve absorption bed and can then be fed to a distillation column to recover dissolved ethylene. Recovered ethylene can be recycled via an ethylene recycle loop while the product is fed to an intermediate vessel, after which the product can be fed to a separation section. In certain embodiments, the linear alpha olefins produced from the reactor can be directed into a separation train.

As illustrated in FIG. 1, the system 10 can include a reactor 12, a toluene (or other solvent) source 14, and a separation train 16. In normal production mode, reactants 18, such as ethylene, solvent, and a catalyst can be fed into the reactor 12 to produce linear alpha olefins and various impurities such as branched olefins and polymeric material. After the reaction, a discharge stream 20 can be directed into the separation train 16, wherein the discharge stream can include unreacted reactants, the produced linear alpha olefins, such as $C_4$-$C_{20+}$ olefins, solvent, catalyst, and various impurities. The separation train 16 can be configured to separate the linear alpha olefins from the solvent, catalyst, various impurities, and any unreacted ethylene. The separation train 16 can separate each linear alpha olefin, for example, yielding a $C_4$ stream, $C_6$ stream, $C_8$ stream, and so on. The separation train 16 can also separate the linear alpha olefins into certain fractions, such as $C_4$-$C_{10}$ fraction, $C_{11}$-$C_{17}$ fraction, $C_{18}$-$C_{20}$ fraction, $C_{20+}$ fractions, or any other desired fraction.

The linear alpha olefin product can be isolated using procedures including aqueous caustic catalyst quench followed by water washing and final product recovery by distillation. For example, the liquid product including the solvent (e.g., toluene) with the dissolved ethylene can be fed to the separation train 16 as noted above. In a first column, the unconsumed ethylene can be separated from the linear alpha olefin product and the solvent. The ethylene can be recycled back to the reactor. The heavier fractions can be routed to the subsequent separation section where the heavier fractions can be divided into the different linear alpha olefin fractions (e.g., $C_8$, $C_{10}$, $>C_{12}$). The solvent can be recovered and also recycled back to the reactor.

Polymer fouling within the reactor can occur during the oligomerization reaction process. Such fouling is typically detected by, for example, reduced effluent flow rates, reduced internal condenser performance, increased differential pressure at various locations within the reactor, and the like. Such fouling can be treated by flushing the reactor with toluene or another solvent to remove polymeric material by-product. The flushed toluene comprising the polymeric material can be directed into a separation train comprising the linear alpha olefin reaction products. The polymeric material is soluble in at least one of the linear alpha olefins, such that the flushed toluene can exit the separation train essentially free of the polymeric material and can be recycled back to the toluene source for subsequent flushing of the reactor.

Purification Process for 1-Hexene

If the desired alpha olefin product in an ethylene oligomerization product stream is 1-hexene having a boiling point of 63.48 °C, one example of a problematic impurity is 2-ethyl-1-butene, which has a boiling point of 64.67 °C. See, for example, a typical 1-hexene product stream composition set forth in Table 1 below.

TABLE 1

| Component | B.P., °C | 1-Hexene Isomers, wt.% |
|---|---|---|
| 2-Ethyl-1-Butene | **63.4** | 0.763 |
| 3-methyl-1-Pentene | 54.0 | 0.00216 |
| n-Hexane | 68.73 | 0.133 |
| Trans-3-Hexene | 67 | 0.049 |
| Cis-3-Hexene | 67 | 0.017 |
| Trans-2-Hexene | 67.9 | 0.261 |
| Cis-2-Hexene | 67.9 | 0.347 |
| Trans 3-Methyl 2-Pentene | 67-68 | 0.00438 |
| Cis 3-Methyl 2-Pentene | 67-68 | 0.00354 |
| 1-Hexene | **63.85** | |

However, 2-ethyl-1-butene can be converted to a higher boiling point isomer such as cis-/trans-3-methyl-2-pentene using an isomerization catalyst. This equilibrium limited reaction is shown below:

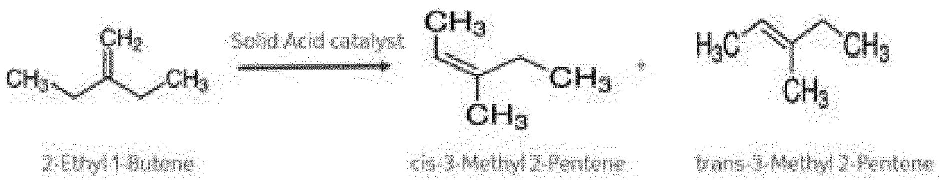


2-Ethyl-1-Butene cis-3-Methyl 2-Pentene trans-3-Methyl 2-Pentene.

According to the present disclosure, in certain embodiments, the separation train 16 of FIG. 1 will include at least one isomerization reactor and at least one distillation column. One example purification system is shown in FIG. 2. As shown, system 100 can include a reactor 110 and a separation unit 112. A first stream 101 containing 1-hexene and 2-ethyl-1-butene can be fed to the reactor 110. In the reactor 110, the first stream 101 can be contacted with an isomerization catalyst to form a second stream 102 containing 1-hexene and 3-methyl-2-pentene. The second stream 102 is typically fed to a separation unit 112. In the separation unit 112, the second stream can be separated to form a third stream containing 1-hexene and a fourth stream containing the 3-methyl-2-pentene.

The feed stream to the reactor 110 can vary in composition, but is typically at least 96 wt.% or at least 98 wt.% 1-hexene (e.g., about 96 wt.% to about 98.5 wt.% or about 98 wt.% to about 98.5 wt.%) and at least 0.5 wt.% or at least 0.8 wt.% 2-ethyl-1-butene (e.g., about 0.5 wt.% to about 1.5 wt.% or about 0.8 wt.% to about 1.2 wt.%).

The reactor 110 can be any suitable reactor, including, but not limited to, a fixed bed reactor, moving bed reactor, trickle bed reactor, rotating bed reactor, slurry reactor, or fluidized bed reactor. In certain embodiments, the reactor 110 can be a fixed bed reactor, and can include a stationary bed containing the isomerization catalyst such that the first stream 101 can be passed through and/or over the stationary bed. In the reactor 110, stream 101 can be contacted with the isomerization catalyst to selectively isomerize 2-ethyl-1-butene in the first stream into 3-methyl-2-pentene.

The first stream 101 can be contacted with the isomerization catalyst at, for example, i) a temperature of 40 °C to 100 °C, or 40 °C to 60 °C, or at least any one of, equal to any one of, or between any two of 40, 42, 44, 46, 48, 50, 52, 54, 56, 58, 60, 65, 70, 75, 80, 85, 90, 95 and 100 °C; ii) a pressure of 0.1 barg to 10 barg or at least any one of, equal to any one of, or between any two of 0.1, 0.2, 0.3, 0.4, 0.5, 1, 2, 3, 4, 5, 6, 7, 8, 9 and 10 barg; and/or iii) a liquid hourly space velocity (LHSV) of 0.5 $h^{-1}$ to 10 $hr^{-1}$, or at least any one of, equal to any one of, or between any two of 0.5, 1, 2, 3, 4, 5, 6, 7, 8, 9 and 10 $hr^{-1}$ or any combinations thereof.

The 2-ethyl-1-butene conversion for the isomerization reaction can be 50 % to 100 % by weight, such as 70 % to 100 %, or 80 % to 99.9 %, or at least any one of, equal to any one of, or between any two of 50, 55, 60, 65, 70, 75, 80, 85, 90, 95, 96, 97, 97.5, 97.6, 97.8, 98, 98.5, 99, 99.5, 99.7, 99.8, 99.9 and 100 %. The total selectivity for 3-methyl-2-pentene for

the isomerization reaction can be 50 % to 100 % by weight, or at least any one of, equal to any one of, or between any two of 50, 55, 60, 65, 70, 75, 80, 85, 90, 95, 99 and 100 % by weight. 1-Hexene conversion during the isomerization reaction can be less than 10 % by weight, or less than 5 %, or less than 3 %, or less than 2.5 %, or less than 2.0%, or less than 1.5 %, or less than 1.0 %, or less than 0.5 %, such as 0.5 % to 10 %, or less than, equal to any one of, or between any two of 0.5, 1, 1.5, 2, 3, 4, 5, 6, 7, 8, 9, and 10 % by weight.

The type of distillation column (e.g., separation unit 112) may vary, with examples including columns with bubble cap trays, valve trays, or sieve trays. Use of an isomerization catalyst as contemplated in this disclosure can reduce the number of stages that might otherwise be required to achieve very high degrees of purity for certain linear alpha olefins, such as 1-hexene. For example, in certain embodiments, the number of distillation stages required to achieve a high degree of linear alpha olefin purity (e.g., a purity of about 99.5 wt.% or greater) is about 150 stages or less or about 120 stages or less (e.g., about 80 to about 150 stages).

The second stream 102 can be separated by distillation in the distillation column to form the third stream 103 containing 1-hexene and the fourth stream 104 containing 3-methyl-2-pentene. The distillation column operation conditions for separation of the second stream 102 can include i) a temperature of 50 °C to 100 °C, or 55 °C to 75 °C, or at least any one of, equal to any one of, or between any two of 50, 55, 60, 65, 70, 75, 80, 85, 90, 95 and 100 °C; and/or ii) a pressure of 0 barg to 3 barg, or at least any one of, equal to any one of, or between any two of 0, 0.01, 0.1, 0.5, 1, 1.5, 2, 2.5 and 3 barg. The boiling points of cis and trans 3-methyl-2-pentene, are different enough from 1-hexene, such that through distillation of the second stream, 3-methyl-2-pentene can be separated from 1-hexane. In certain embodiments, the third stream 103 can be formed as the top distillate of the distillation column and the fourth stream 104 can be formed as the bottom distillate of the distillation column.

Alternatively, instead of using a catalyst bed upstream of a distillation column as shown in FIG. 2, external side reactors containing the catalyst can be positioned to receive a feed stream from a stage of the distillation column and the effluent from the side reactor can be fed back to a stage of the distillation column. One or more side reactors can be operated, for example, as either plug flow reactors (otherwise known as tubular reactors) or continuous stirred-tank reactors (CSTR), and can be operated at temperatures and pressures that are independent of the distillation column operating temperature and pressure. The 1-hexene feed containing impurities can be fed to a tubular reactor in either upflow or downflow mode.

## Isomerization Catalyst

The isomerization catalyst can selectively isomerize 2-ethyl-1-butene, in the presence of 1-hexene (and hexane and other isomer(s) of 1-hexene, if present), to form 3-methyl-2-pentene. In some embodiments, the catalyst material is an activated alumina material, such as high bulk density gamma-alumina, low or medium bulk density large pore gamma-alumina, and low bulk density large pore boehmite and gamma-alumina.

High surface area alumina materials, also sometimes referred to as "gamma alumina" or "activated alumina," typically exhibit a BET surface area in excess of 60 $m^2/g$, often up to about 200 $m^2/g$ or higher. Such activated alumina is usually a mixture of the gamma and delta phases of alumina, but may also contain substantial amounts of eta, kappa and theta alumina phases. "BET surface area" has its usual meaning of referring to the Brunauer, Emmett, Teller method for determining surface area by $N_2$ adsorption.

In certain embodiments, the alumina material has i) a BET surface area of 200 $m^2/g$ to 550 $m^2/g$ or at least any one of, equal to any one of, or between any two of 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540 and 550 $m^2/g$; ii) an average particle size of 1 mm to 8 mm or at least any one of, equal to any one of, or between any two of 1 , 2, 3, 4, 5, 6, 7 and 8 mm; and/or iii) an average crush strength of 0.5 to 40 kg, or at least any one of, equal to any one of, or between any two of 0.5, 1, 5, 10, 15, 20, 25, 30, 35 and 40 kg, or any combination thereof.

Certain useful alumina materials are commercially available as alumina materials chemically modified to increase the presence of acidic functional groups. A "modified alumina" as used herein refers to an alumina material chemically modified to enhance the acid functionality of the alumina. A non-limiting example of a commercially available modified alumina is SELEXSORB® CD available from BASF Corporation. Further, there are known processes for preparing $\gamma$-$Al_2O_3$ with rich Brønsted acid sites and reduced Lewis acid sites using a sol-gel method with $NH_4BF_4$ as a modifier. See J. Phys. Chem. C 2014, 118, 12, 6226-6234.

The alumina material utilized in the present disclosure is characterized by the presence of sufficient Brønsted acid sites for adsorption and isomerization of 2-ethyl-1-butene. For example, in certain embodiments, the alumina material will have at least 0.01 mmol/g catalyst of Brønsted acid sites (e.g., at least 0.02 or at least 0.03 or at least 0.04 mmol/g), but typically less than 0.5 mmol/g catalyst of Brønsted acid sites (e.g., less than 0.4 or less than 0.3 or less than 0.2 or less than 0.1 or less than 0.09 mmol/g). If the catalyst contains a high density of Brønsted acid sites, the catalyst is more prone to non-selective isomerization, which could result in undesirable 1-hexene conversion. Concentration of Brønsted acid sites can be measured by FTIR spectroscopy using pyridine as probe molecule, as set forth in the Experimental. The nature of

the Bronsted acid sites of the modified alumina catalyst is that they are weak in strength, that is, the pyridine probe molecules held by the Bronsted sites can be desorbed under vacuum at about or above 150 °C.

As noted above, in certain embodiments, the isomerization catalyst can be characterized based on how much of the undesirable first isomer is converted to the second isomer. For example, in certain embodiments, the isomerization catalyst is capable of converting about 80 wt.% or greater of 2-ethyl-1-butene (e.g., about 85 wt.% or greater or about 90 wt.% or greater or about 95 wt.% or greater) to the second isomer in about 1 hour at a pressure of about 0.1-10 barg, a temperature of about 40 - 100 °C, and a Liquid Hourly Space Velocity of about 0.5 - 10.0 $h^{-1}$. In some embodiments, about 3 wt.% or less of 1-hexene (e.g., about 2 wt.% or less or about 1 wt.% or less) is converted to a different isomer under the same reaction conditions noted above

Use of an isomerization catalyst as contemplated herein can enable purification of 1-hexene or other linear alpha olefins to a purity of about 99.5 wt.% or greater in the distillation column overhead stream. In certain embodiments, the overhead product stream from the distillation column is characterized by very low impurity content, such as about 0.3 wt.% or less, or about 0.2 wt.% or less, or about 0.15 wt.% or less of branched olefins, such as 2-ethyl-1-butene. Where the target linear alpha olefin is 1-hexene, in certain embodiments, the overhead product stream is also characterized by very low n-hexane content, such as about 200 ppm or less or about 150 ppm or less n-hexane.

<u>Regeneration of Catalyst</u>

It has been surprisingly discovered that periodically regenerating the modified alumina catalyst as set forth herein can lead to strong catalyst performance that, in certain embodiments, is sustainable over thousands of hours. Typically, the regenerating process will involve stopping the linear alpha olefin product stream feed to the catalytic reactor and introducing a non-oxidizing environment to the reactor at time on stream intervals of no more than about 200 hours. In certain embodiments, the reactor is subjected to the non-oxidizing environment for a time period of about 15 hours or greater and the non-oxidizing environment has a temperature of about 250 °C or greater for at least a portion of the time period. As set forth in the Experimental below, such a regeneration process can be used repeatedly to return the catalyst to the same performance level as fresh catalyst.

The time interval between regeneration steps can vary, and will depend in part on the moisture level in the feed stream. Higher levels of moisture in the feed will often necessitate shorter intervals between regeneration processes. For example, a low moisture feed having less than about 10 ppm water could enable longer intervals between regeneration steps, such as greater than about 120, or greater than about 140, or greater than about 160, or greater than about 180 hours as the interval period (e.g., about 150 to about 300 hours or even higher). Conversely, higher levels of moisture in the feed (e.g., about 30 ppm or higher such as about 30 to about 80 ppm) may lead to shorter intervals, such as less than about 150 hours, or less than about 130 hours, or less than about 110 hours, or less than about 100 hours (e.g., about 80 to about 150 hours).

The non-oxidizing environment is typically any environment that is inert to the modified alumina catalyst. Examples include a vacuum environment or an inert gas environment, such as nitrogen or a noble gas environment. The non-oxidizing environment will be substantially or completely free of oxygen.

In certain embodiments, the regeneration process utilizing the non-oxidizing environment will be conducted in sequential stages. For example, the regeneration process can include a first lower temperature stage that comprises passing an inert gas through the reactor at an inert gas temperature of about 200 °C or less for a time period of about 2 hours or greater. This lower temperature regeneration step is useful to purge water and any residual physisorbed hydrocarbons from the catalyst bed.

In certain embodiments, the lower temperature stage can include passing the inert gas through the reactor at an inert gas temperature of about 150 °C or less, such as about 40 °C to about 150 °C. The lower temperature stage itself may contain multiple stages at different temperatures, such as two or three temperature levels within the above-noted temperature range. The total regeneration time for the lower temperature stage can vary, but is typically about 4 hours or greater. In certain embodiments, the total regeneration time for the lower temperature stage is about 2 to about 6 hours or about 4 to about 6 hours. Higher temperatures usually enable shorter time periods for this regeneration step. Accordingly, one of skill can elect to use lower temperatures combined with longer time periods or opt for higher temperatures and shorter time periods within the ranges noted above. The inert gas flow rate during this stage is typically about 120 to about 300 $cm^3$/min, such as about 150 to about 250 $cm^3$/min.

In certain embodiments, the regeneration process can include a second higher temperature stage, such as a stage that comprises passing the inert gas through the reactor at an inert gas temperature of about 250 °C or greater for a time period of about 8 hours or greater. This higher temperature step can advantageously provide further removal of adsorbed compounds on the Brønsted acid sites.

In certain embodiments, the higher temperature stage can include passing the inert gas through the reactor at an inert gas temperature of about 300 °C or less, such as an inert gas temperature of about 250 °C to about 300 °C. The higher temperature stage itself may contain multiple stages at different temperatures, such as two or three temperature levels

within the above-noted temperature range. The total regeneration time for the higher temperature stage can vary, but is typically about 14 hours or less. In certain embodiments, the total regeneration time for the lower temperature stage is about 8 to about 14 hours or about 10 to about 12 hours. Higher temperatures usually enable shorter time periods for this regeneration step. Accordingly, one of skill can elect to use lower temperatures combined with longer time periods or opt for higher temperatures and shorter time periods within the ranges noted above. The inert gas flow rate during this stage is typically about 120 to about 300 $cm^3$/min, such as about 150 to about 250 $cm^3$/min.

The non-oxidizing environment may be insufficient to fully regenerate the catalyst after a certain number of hours on stream due to build-up of carbon/coke on the catalyst surface. Accordingly, in certain embodiments, the regeneration process further includes periodically introducing an oxidizing gas to the reactor, such as air or purified oxygen. In certain embodiments, oxidizing gas is introduced at a gas temperature of about 300 °C to about 450 °C for a time period of 1 hour to 24 hours or longer (e.g., at least 1 hour or longer or at least 10 hours or longer or at least 15 hours or longer or at least 20 hours or longer). The regeneration time to remove carbonaceous species is dependent on the temperature and concentration of oxygen. The regeneration under oxidative environment can be applied when the regeneration under non-oxidative environment is not improving the catalyst performance comparable to fresh catalyst. That is, in a plant operation, such oxidative regeneration can be applied when the catalyst performance after non-oxidative regeneration declines quickly to a predetermined threshold level of performance, such as defined by conversion selectivity or cycle length.

It is advantageous to avoid subjecting the catalyst material to abrupt temperature changes during the regeneration process, which could lead to degradation of the catalyst structure. Thus, when it is desired to change the regeneration temperature, such changes are typically achieved using relatively small rates of temperature change, such as temperature increases or decreases at a rate of about 1 to about 5 °C/min.

EXPERIMENTAL

The following examples utilize Selexsorb® CD catalyst commercially available from BASF Corporation. Table 2 below provides certain characteristics of the catalyst material.

TABLE 2

| Commercial Name | Selexsorb® CD |
|---|---|
| **Chemical Composition (wt.%)** | $Al_2O_3$ 95.1% (plus proprietary modifier) LOI (250-1100 °C) 4.5% |
| **Surface Area, $m^2$/g** | 420 |
| **Crush Strength, lb (kg)** | 10 (5) |
| **Bulk Density, lbs/$ft^3$ (kg/$m^3$)** | 43 (689) |
| **Acidity[1]** | Bronsted acid site 0.12 mmol/g catalyst Lewis acid site 0.80 mmol/g catalyst |

[1]Measured by FTIR spectroscopy using pyridine as probe molecule

The FTIR spectroscopy measurement process used was as follows. The alumina catalyst was ground into fine powder and pressed (under 10 ton pressure) into a self-supported thin wafer of 0.65 cm diameter (having a surface area of 1.33 $cm^2$) and a weight of 0.02 g (i.e., 0.015 g/$cm^2$). The sample wafer was heated to a maximum of 280 °C under vacuum or flow of $N_2$ (depending on fresh or spent catalyst) for 4 to 24 h. The catalyst wafer was cooled and exposed to pyridine vapor at 100 °C for 30 min and then desorbed at 100 °C under vacuum. Infrared spectra of pretreated and pyridine adsorbed sample were recorded at room temperature as an average of 16 runs at 4 $cm^{-2}$ resolution using Perkin-Elmer Spectrum One FTIR Spectrometer.

To determine acid sites, the Beer-Lambert-Bouguer law was applied in the form of

$$A = \varepsilon * Cs$$

where, Cs is concentration of Bronsted acid sites in μmol/g, A denotes the integrated absorbance of the corresponding infrared band in the unit cm/g, which is normalized to catalyst wafer density, and ε is the integrated molar absorption coefficients (see reference Gabrienko, A.A., et al., Journal of Physical Chemistry, 122, p25386, 2018). The ε values of 1.67 cm/μmol and 2.22 cm/μmol for pyridine adsorbed on Brønsted acid sites and on Lewis acid sites were used from reference Emeis, C.A., Journal of Catalysis, 141, p347, 1993.

Example 1

Selexsorb® CD catalyst (size 1.4-1.7 mm, full sphere, 17.56 g, 25 $cm^3$) was loaded in 1-inch OD stainless steel tubular reactor, and glass inserts were added at both ends of the catalyst bed. Catalyst was dried under $N_2$ flow (170 $cm^3$/min) at 45°C for 2 h, then heating at 5°C/min to 150°C and held for 2 h, then heating at 5°C/min to 280°C and held for 12 h. The catalyst bed temperature was cool down to 45°C and $N_2$ flow was stopped for introduction of feed to the reactor.

1-Hexene feed was dried by using Molecular Sieve 4A. The catalytic test was started by flowing 1-hexene feed at about 0.4 mL/min (feed rates varied at the start up, rates are shown in FIG. 3), catalyst bed temperature was 45°C and inlet pressure was 1.5 psig.

Table 3 below shows feed and product stream (at time on stream 56.3 h) compositions. 2E1B and 1-H conversions were found to be 98.73% and 0.09%. 2E1B was mostly converted to cis- and trans-3-methyl-2-pentene.

2E1B conversion decreased with time on stream. The catalyst was regenerated by first purging the catalyst bed flowing $N_2$ (170 $cm^3$/min) at 45 °C for 2 h, then heating at 5°C/min to 150 °C and held for 2 h, then heating at 5°C/min to 280 °C and held for 12 h before cooling to 45 °C for re-introduction of feed to the reactor. Total regeneration time was 16 hours with three temperature steps under $N_2$ flow of 170 $cm^3$/min.

The regeneration process was repeated periodically as catalyst performance declined. Most of the regeneration steps in this example occurred after 300 hours of time on stream. As shown graphically in FIG. 3, catalyst performance declined over time and the periodic regeneration process was unable to return the catalyst to its original performance level.

TABLE 3. Feed and Product Stream Composition

| **Composition[1]** | | **wt.%** | |
|---|---|---|---|
| | | Feed Stream | Product Stream[2] |
| **3-methyl-1-pentene** | | 0.019 | 0.005 |
| **1-Hexene (1-H)** | | 98.370 | 98.281 |
| **2-Ethyl-1-butene (2E1B)** | | 1.026 | 0.013 |
| **Hexane** | | 0.045 | 0.123 |
| **Trans-3-hexene** | | 0.028 | 0.037 |
| **Cis-3-hexene** | | 0.010 | 0.015 |
| **Trans-2-hexene** | | 0.212 | 0.238 |
| **Cis-3-methyl-2-pentene** | | 0.009 | 0.318 |
| **Cis-2-hexene** | | 0.275 | 0.332 |
| **Trans-3-methyl-2-pentene** | | 0.006 | 0.638 |
| **Conversions** | 2E1B | | 98.73% |
| | 1-H | | 0.09% |
| [1]Feed contained 60-70 ppm water; [2]At time on stream 56.3 hours | | | |

Example 2

The experiment of Example 1 was repeated with a slightly different catalyst loading (Selexsorb® CD; 24.34 g; 36 $cm^3$) and using the same catalyst drying process of Example 1.

Compared to catalyst regeneration in Example 1, a longer duration of $N_2$ flow with an addition of a step was used in Example 2. The catalyst was regenerated by first purging the catalyst bed by flowing $N_2$ (170 $cm^3$/min) at 45 °C for 2 h, then heating at 5 °C/min to 60 °C and held for 12 h, then heating at 5 °C/min to 150 °C and held for 4 h, then heating at 5 °C/min to 280°C and held for 12 h before cooling to 45 °C for re-introduction of feed to the reactor. Total regeneration time was about 30 hours with four temperature steps under $N_2$ flow of 170 $cm^3$/min.

Table 4 below shows feed and product stream (at time on stream 126.6 h) compositions. 2E1B and 1-H conversions were found to be 97.47% and 0.04%. 2E1B was mostly converted to cis- and trans-3-methyl-2-pentene.

In Example 2, the catalyst life increased with the increase of duration of $N_2$ flow with an addition of a step and shorter duration (relative to Example 1) between the regenerations. As shown graphically in FIG. 4, catalyst performance was maintained over time. Most of the regeneration steps occurred after no more than 200 hours of time on stream.

TABLE 4. Feed and Product Stream Composition

| Composition[1] | | wt.% | |
|---|---|---|---|
| | | Feed Stream | Product Stream[2] |
| **3-methyl-1-pentene** | | 0.070 | 0.082 |
| **1-Hexene** | | 98.662 | 98.626 |
| **2-Ethyl-1-butene** | | 0.995 | 0.025 |
| **Hexane** | | 0.003 | 0.031 |
| **Trans-3-hexene** | | 0.019 | 0.020 |
| **Cis-3-hexene** | | 0.007 | 0.005 |
| **Trans-2-hexene** | | 0.086 | 0.100 |
| **Cis-3-methyl-2-pentene** | | 0.061 | 0.360 |
| **Cis2-hexene** | | 0.019 | 0.031 |
| **Trans-3-methyl-2-pentene** | | 0.078 | 0.719 |
| **Conversions** | 2E1B | | 97.49% |
| | 1-H | | 0.04% |
| [1]Feed contained 40-70 ppm water; [2]At time on stream 126.6 hours | | | |

Example 3

Selexsorb® CD catalyst (size 2.8-3.2 mm, full sphere, 6.1 g, 9 $cm^3$) was loaded in 0.5-inch OD stainless steel tubular reactor, and glass inserts were added at both ends of the catalyst bed. Catalyst was dried under $N_2$ flow (200 $cm^3$/min) at 45 °C for 2 h, then heating at 2 °C/min to 150 °C and held for 2 h, then heating at 2°C/min to 280 °C and held for 12 h. The catalyst bed temperature was cool down to 45°C and $N_2$ flow was stopped for introduction of feed to the reactor.

1-Hexene feed was dried by using Nitrogen gas purging for 0.5 h. The catalytic test was started by flowing 1-hexene feed at about 0.1 mL/min, catalyst bed temperature was 45°C and inlet pressure was 1.5 psig. Table 5 below shows feed and product stream (at time on stream 192 h) compositions. 2E1B and 1-H conversions were found to be 97.72% and 0.01%. 2E1B was mostly converted to cis- and trans-3-methyl-2-pentene.

2-Ethyl 1-butene conversion decreased with time on stream. The catalyst was periodically regenerated by first purging the catalyst bed flowing $N_2$ (200 $cm^3$/min) at 45 °C for 2 h, then heating at 2 °C/min to 150°C and held for 2 h, then heating at 2 °C/min to 280 °C and held for 12 h before cooling to 45 °C for re-introduction of feed to the reactor. Total $N_2$ regeneration time was about 16 hours with three temperature steps under $N_2$ flow of 200 $cm^3$/min. The catalyst was also subjected to an air regeneration step once during the experiment at the 1924 hour mark. The air regeneration consisted of (a) stopping 1-hexene feed stream and passing $N_2$ gas to purge hydrocarbon for about an hour, (b) flowing air at 200 $cm^3$/min and raising catalyst bed temperature (5 °C/min) to 110 °C and holding for 2 hours, (c) increasing temperature (1 °C/min) to 350 °C and holding for 5 hours. The catalyst bed temperature was cool down to room temperature and purge $N_2$ before catalytic testing. The air regeneration step was able to return the catalyst to the same performance level as fresh catalyst after catalyst performance began to degrade following repeated $N_2$ regeneration.

In Example 3, the catalyst life increased with frequent catalyst $N_2$ regeneration (relative to Example 1). As shown graphically in FIG. 5, catalyst performance was maintained over time, with no appreciable drop in performance (with regular regeneration) for more than 2500 hours. Most of the regeneration steps occurred after no more than 200 hours of time on stream. Table 6 below provides the frequency and type of regeneration used at each regeneration step.

TABLE 5. Feed and Product Stream Composition

| Composition[1] | wt.% | |
|---|---|---|
| | Feed Stream | Product Stream[2] |
| **3-methyl-1-pentene** | **0.05** | **0.072** |
| **1-Hexene** | **98.3** | 98.29 |
| **2-Ethyl-1-butene** | **0.922** | 0.021 |
| **Hexane** | **0.003** | 0.011 |

(continued)

| Composition[1] | wt.% | | |
|---|---|---|---|
| | Feed Stream | Product Stream[2] | |
| **Trans-3-hexene** | **0.019** | 0.02 | |
| **Cis-3-hexene** | **0.007** | 0.01 | |
| **Trans-2-hexene** | **0.16** | 0.15 | |
| **Cis-3-methyl-2-pentene** | **0.046** | 0.701 | |
| **Cis2-hexene** | **0.019** | 0.02 | |
| **Trans-3-methyl-2-pentene** | | **0.48** | 0.705 |
| **Conversions** | 2E1B | | 97.72% |
| | 1-H | | 0.01% |
| [1]Feed contained 55-70 ppm water; [2]At time on stream 192 hours | | | |

TABLE 6. Regeneration Schedule

| Regeneration Type | After Time on Stream |
|---|---|
| $N_2$ regeneration | 144 |
| $N_2$ regeneration | 288 |
| $N_2$ regeneration | 408 |
| $N_2$ regeneration | 504 |
| $N_2$ regeneration | 648 |
| $N_2$ regeneration | 768 |
| $N_2$ regeneration | 888 |
| $N_2$ regeneration | 1008 |
| $N_2$ regeneration | 1128 |
| $N_2$ regeneration | 1248 |
| $N_2$ regeneration | 1368 |
| $N_2$ regeneration | 1488 |
| $N_2$ regeneration | 1632 |
| $N_2$ regeneration | 1752 |
| $N_2$ regeneration | 1872 |
| $O_2$ regeneration | 1924 |
| $N_2$ regeneration | 2044 |
| $N_2$ regeneration | 2164 |
| $N_2$ regeneration | 2284 |
| $N_2$ regeneration | 2404 |
| $N_2$ regeneration | 2524 |
| $N_2$ regeneration | 2644 |
| $N_2$ regeneration | 2764 |
| $N_2$ regeneration | 2884 |
| $N_2$ regeneration | 3004 |
| $N_2$ regeneration | 3124 |

(continued)

| Regeneration Type | After Time on Stream |
|---|---|
| $N_2$ regeneration | 3244 |
| $N_2$ regeneration | 3364 |

Example 4

In a separate experiment, a fresh sample of Selexsorb® CD was analyzed using FTIR spectroscopy using pyridine as probe molecule to measure Bronsted and Lewis acid sites. Spent catalyst from Example 2, after catalytic testing for about 850 hours with frequent $N_2$ regeneration at 280 °C, was also subjected to the same acid site analysis as a comparison. The results are set forth in Table 7 below. Each sample was pretreated as indicated in Table 7 prior to analysis. As shown, the longer $N_2$ treatment of the spent catalyst was able to recover the Brønsted acid sites, which is consistent with the results set forth in Examples 1-3.

TABLE 7

| Catalyst and Pretreatment Condition | Brønsted acid site, μmol/g | Lewis acid site, μmol/g | Total acid site, μmol/g |
|---|---|---|---|
| Selexsorb® CD fresh (under vacuum, 70°C 4 h, 150°C 4 h, 280°C 4h) | 122 | 795 | 917 |
| Selexsorb® CD Spent (under vacuum, 70 °C 4 h, 150 °C 4 h, 280°C 4h) | 31 | 506 | 537 |
| Selexsorb® CD Spent (under $N_2$ flow, 70 °C 4 h, 150 °C 4 h, 280 °C 24h) | 146 | 509 | 655 |

Comparative Example 1

The experiment of Example 1 was repeated using different alumina catalysts: Selexsorb® CDL available from BASF Corporation and Actisorb® 100-1 available from Clariant Ltd. Unlike Selexsorb® CD, neither of these adsorbents are believed to have Bronsted acid sites within the ranges set forth in the present disclosure.

Selexsorb® CDL and Actisorb® 100-1 were catalytically tested using the same catalyst pretreatment and test conditions as described in Example 1. The results are provided in Table 8 below. A shown, neither alumina-based catalyst was able to convert a significant amount of 2E1B to cis- and trans-3-methyl-2-pentene.

TABLE 8

| Selexsorb® CDL | | | Actisorb® 100-1 | | |
|---|---|---|---|---|---|
| Hours on stream | % 2E1B Conv. | % 1-H Conv. | Hours on stream | % 2E1B Conv. | % 1-H Conv. |
| 0.72 | 21.6 | 0 | 0.74 | 6.12 | 0.3 |
| 1.45 | 17.5 | 0 | 1.47 | 1.42 | 0 |
| 2.17 | 16.2 | 0 | 2.21 | 1.06 | 0 |
| 2.91 | 17.2 | 0 | 2.94 | 1.46 | 0 |

Comparative Example 2

An unmodified gamma alumina was analyzed using FTIR spectroscopy using pyridine as probe molecule to measure Brønsted and Lewis acid sites as described above. The FTIR spectrum is set forth in FIG. 6, and shows a peak at 1450 $cm^{-1}$ (Lewis acid site), but no peak at around 1545 $cm^{-1}$ (characteristic peak for Brønsted acid site). This is consistent with the belief that the presence of Brønsted acid sites is important for strong 2E1B conversion performance.

In general, the invention may alternately comprise, consist of, or consist essentially of, any appropriate components herein disclosed. The invention may additionally, or alternatively, be formulated so as to be devoid, or substantially free, of any components, materials, ingredients, adjuvants or species used in the prior art compositions or that are otherwise not necessary to the achievement of the function and/or objectives of the present invention.

Many modifications and other implementations of the disclosure will come to mind to one skilled in the art to which this disclosure pertains having the benefit of the teachings presented in the foregoing descriptions and the associated

figures. Therefore, it is to be understood that the disclosure is not to be limited to the specific implementations disclosed herein and that modifications and other implementations are intended to be included within the scope of the appended claims. Although specific terms are employed herein, they are used in a generic and descriptive sense only and not for purposes of limitation.

## Claims

1. A catalytic process for selective isomerization of 2-ethyl-1-butene in a linear alpha olefin product stream, comprising:

   feeding a linear alpha olefin product stream comprising at least one linear alpha olefin, such as 1-hexene, and 2-ethyl-1-butene to a reactor housing a modified alumina catalyst to isomerize at least a portion of the 2-ethyl-1-butene to cis- or trans-3-methyl-2-pentene, the period during which said feeding occurs being time on stream;
   withdrawing an effluent from the reactor containing less 2-ethyl-1-butene than the linear alpha olefin product stream; and
   periodically regenerating the modified alumina catalyst by stopping the feeding step and introducing a non-oxidizing environment to the reactor at time on stream intervals of no more than about 200 hours, wherein the reactor is subjected to the non-oxidizing environment for a time period of about 15 hours or greater and wherein the non-oxidizing environment has a temperature of about 250 °C or greater for at least a portion of the time period.

2. The catalytic process of claim 1, wherein the non-oxidizing environment comprises a vacuum environment or an inert gas environment, such as nitrogen or a noble gas environment.

3. The catalytic process of claim 1 or 2, wherein the regenerating comprises passing an inert gas through the reactor in two sequential stages comprising i) a first lower temperature stage comprising passing the inert gas through the reactor at an inert gas temperature of about 200 °C or less for a time period of about 2 hours or greater; and ii) a second higher temperature stage comprising passing the inert gas through the reactor at an inert gas temperature of about 250 °C or greater for a time period of about 8 hours or greater.

4. The catalytic process of claim 3, wherein the first lower temperature stage comprises one or more of:

   passing the inert gas through the reactor at an inert gas temperature of about 150 °C or less;
   passing the inert gas through the reactor at an inert gas temperature of about 40 °C to about 150 °C;
   passing the inert gas through the reactor at multiple inert gas temperatures ranging from about 40 °C to about 150 °C, wherein the inert gas temperature is increased to each of the multiple inert gas temperatures at a rate of about 1 to about 5 °C/min;
   passing the inert gas through the reactor for a time period of about 4 hours or greater; and
   passing the inert gas through the reactor for a time period of about 2 to about 6 hours.

5. The catalytic process of claim 3, wherein the second higher temperature stage comprises one or more of:

   passing the inert gas through the reactor at an inert gas temperature of about 300 °C or less;
   passing the inert gas through the reactor at an inert gas temperature of about 250 °C to about 300 °C;
   passing the inert gas through the reactor for a time period of about 14 hours or less; and
   passing the inert gas through the reactor for a time period of about 8 to about 12 hours.

6. The catalytic process of any one of claims 3 to 5, wherein the transition from the first lower temperature stage to the second higher temperature stage occurs at a rate of about 1 to about 5 °C/min.

7. The catalytic process of any one of claims 3 to 6, wherein the inert gas flow rate is about 120 to about 300 $cm^3$/min.

8. The catalytic process of any one of claims 1 to 7, further comprising periodically regenerating the modified alumina catalyst by stopping the feeding step and introducing an oxidizing gas to the reactor.

9. The catalytic process of claim 8, wherein the oxidizing gas comprises oxygen at a gas temperature of about 300 °C to about 450 °C, optionally introducing the oxidizing gas for a time period of 1 to 24 hours.

**10.** The catalytic process of any one of claims 1 to 9, wherein the modified alumina catalyst is capable of converting about 80 wt.% or greater of 2-ethyl-1-butene to cis- or trans-3-methyl-2-pentene at a pressure of about 0.1 - 10 barg, a temperature of about 40 - 100 °C, and a Liquid Hourly Space Velocity of about 0.5 - 10 $h^{-1}$, and wherein about 3 wt.% or less of 1-hexene is converted to a different isomer under the same reaction conditions.

**11.** The catalytic process of any one of claims 1 to 10, further comprising feeding the effluent from the reactor to a distillation column to produce an overhead stream comprising the linear alpha olefin and a bottoms stream comprising cis- or trans-3-methyl-2-pentene.

**12.** The catalytic process of claim 11, wherein the overhead stream comprises about 99.5 wt.% 1-hexene or greater and about 0.15 wt.% of 2-ethyl-1-butene or an isomer thereof or less.

**13.** The catalytic process of any one of claims 1 to 12, wherein the modified alumina catalyst has about 0.01 to about 0.5 mmol/g catalyst of Brønsted acid sites as determined by FTIR spectroscopy using pyridine as probe molecule.

**14.** The catalytic process of claim 13, wherein the modified alumina catalyst has about 0.04 to about 0.2 mmol/g catalyst of Brønsted acid sites as determined by FTIR spectroscopy using pyridine as probe molecule.

## Patentansprüche

**1.** Katalytisches Verfahren zur selektiven Isomerisierung von 2-Ethyl-1-buten in einem Produktstrom von linearen α-Olefinen, umfassend:

Zuführen eines Produktstroms von linearen α-Olefinen, der mindestens ein lineares α-Olefin, wie z. B. 1-Hexen, und 2-Ethyl-1-buten umfasst, zu einem Reaktor, der einen Katalysator aus modifiziertem Aluminiumoxid aufnimmt, um zumindest einen Teil von dem 2-Ethyl-1-buten zu cis-3-Methyl-2-penten oder trans-3-Methyl-2-penten zu isomerisieren, wobei die Dauer, während der das Zuführen erfolgt, der Laufzeit entspricht;
Abführen eines Abflusses aus dem Reaktor, der weniger 2-Ethyl-1-buten als der Produktstrom von linearen α-Olefinen enthält; und
regelmäßiges Regenerieren des Katalysators aus modifiziertem Aluminiumoxid durch Beenden des Zuführungsschrittes und Einbringen einer nicht oxidierenden Umgebung in den Reaktor in Laufzeitintervallen von nicht mehr als etwa 200 Stunden, wobei der Reaktor der nicht oxidierenden Umgebung über eine Zeitdauer von etwa 15 Stunden oder größer ausgesetzt wird und wobei die nicht oxidierende Umgebung eine Temperatur von etwa 250 °C oder höher über zumindest einen Teil der Zeitdauer aufweist.

**2.** Katalytisches Verfahren nach Anspruch 1, wobei die nicht oxidierende Umgebung eine Vakuumumgebung oder eine Inertgasumgebung, wie Stickstoff oder eine Edelgasumgebung, umfasst.

**3.** Katalytisches Verfahren nach Anspruch 1 oder 2, wobei das Regenerieren das Hindurchleiten eines Inertgases durch den Reaktor in zwei aufeinanderfolgenden Stufen umfasst, die umfassen: i) eine erste Stufe mit niedrigerer Temperatur, umfassend das Hindurchleiten des Inertgases durch den Reaktor bei einer Temperatur des Inertgases von etwa 200 °C oder geringer über eine Zeitdauer von etwa 2 Stunden oder größer; und ii) eine zweite Stufe mit höherer Temperatur, umfassend das Hindurchleiten des Inertgases durch den Reaktor bei einer Temperatur des Inertgases von etwa 250 °C oder höher über eine Zeitdauer von etwa 8 Stunden oder größer.

**4.** Katalytisches Verfahren nach Anspruch 3, wobei die erste Stufe mit niedrigerer Temperatur eines oder mehrere von Folgendem umfasst:

Hindurchleiten des Inertgases durch den Reaktor bei einer Temperatur des Inertgases von etwa 150 °C oder geringer;
Hindurchleiten des Inertgases durch den Reaktor bei einer Temperatur des Inertgases von etwa 40 °C bis etwa 150 °C;
Hindurchleiten des Inertgases durch den Reaktor bei mehreren Temperaturen des Intertgases, die von etwa 40 °C bis etwa 150 °C reichen, wobei die Temperatur des Inertgases auf jede der mehreren Temperaturen des Inertgases mit einer Geschwindigkeit von etwa 1 bis etwa 5 °C/min erhöht wird;
Hindurchleiten des Inertgases durch den Reaktor über eine Zeitdauer von etwa 4 Stunden oder größer; und
Hindurchleiten des Inertgases durch den Reaktor über eine Zeitdauer von etwa 2 bis etwa 6 Stunden.

5. Katalytisches Verfahren nach Anspruch 3, wobei die zweite Stufe mit höherer Temperatur eines oder mehrere von Folgendem umfasst:

Hindurchleiten des Inertgases durch den Reaktor bei einer Temperatur des Inertgases von etwa 300 °C oder geringer;
Hindurchleiten des Inertgases durch den Reaktor bei einer Temperatur des Inertgases von etwa 250 °C bis etwa 300 °C;
Hindurchleiten des Inertgases durch den Reaktor über eine Zeitdauer von etwa 14 Stunden oder weniger; und
Hindurchleiten des Inertgases durch den Reaktor über eine Zeitdauer von etwa 8 bis etwa 12 Stunden.

6. Katalytisches Verfahren nach einem der Ansprüche 3 bis 5, wobei der Übergang von der ersten Stufe mit niedrigerer Temperatur zu der zweiten Stufe mit höherer Temperatur mit einer Geschwindigkeit von etwa 1 bis etwa 5 °C/min erfolgt.

7. Katalytisches Verfahren nach einem der Ansprüche 3 bis 6, wobei der Durchfluss des Inertgases etwa 120 bis etwa 300 $cm^3$/min beträgt.

8. Katalytisches Verfahren nach einem der Ansprüche 1 bis 7, weiterhin umfassend das regelmäßige Regenerieren des Katalysators aus modifiziertem Aluminiumoxid durch Beenden des Zuführungsschrittes und das Einbringen eines oxidierenden Gases in den Reaktor.

9. Katalytisches Verfahren nach Anspruch 8, wobei das oxidierende Gas Sauerstoff bei einer Temperatur des Gases von etwa 300 °C bis etwa 450 °C umfasst, optional umfassend das Einbringen des oxidierenden Gases über eine Zeitdauer von 1 bis 24 Stunden.

10. Katalytisches Verfahren nach einem der Ansprüche 1 bis 9, wobei der Katalysator aus modifiziertem Aluminiumoxid imstande ist, etwa 80 Gew.-% oder größer von 2-Ethyl-1-buten in cis-3-Methyl-2-penten oder trans-3-Methyl-2-penten bei einem Druck von etwa 0,1-10 barg, einer Temperatur von etwa 40-100 °C und einer stündlichen Raumgeschwindigkeit der Flüssigkeit von etwa 0,5-10 $h^{-1}$ umzuwandeln, und wobei unter den gleichen Reaktionsbedingungen etwa 3 Gew.-% oder weniger von 1-Hexen in ein anderes Isomer umgewandelt werden.

11. Katalytisches Verfahren nach einem der Ansprüche 1 bis 10, weiterhin umfassend das Zuführen des Abflusses aus dem Reaktor zu einer Destillationskolonne zum Erzeugen eines Kopfproduktstroms, der das lineare $\alpha$-Olefin umfasst, und eines Sumpfproduktstroms, der cis-3-Methyl-2-penten oder trans-3-Methyl-2-penten umfasst.

12. Katalytisches Verfahren nach Anspruch 11, wobei der Kopfproduktstrom etwa 99,5 Gew.-% 1-Hexen oder größer und etwa 0,15 Gew.-% von 2-Ethyl-1-buten oder einem Isomer davon oder weniger umfasst.

13. Katalytisches Verfahren nach einem der Ansprüche 1 bis 12, wobei der Katalysator aus modifiziertem Aluminiumoxid etwa 0,01 bis etwa 0,5 mmol/g Katalysator an Brønsted-Säure-Zentren aufweist, wie mittels FTIR-Spektroskopie unter Verwendung von Pyridin als Sondenmolekül bestimmt.

14. Katalytisches Verfahren nach Anspruch 13, wobei der Katalysator aus modifiziertem Aluminiumoxid etwa 0,04 bis etwa 0,2 mmol/g Katalysator an Brønsted-Säure-Zentren aufweist, wie mittels FTIR-Spektroskopie unter Verwendung von Pyridin als Sondenmolekül bestimmt.

## Revendications

1. Procédé catalytique pour l'isomérisation sélective de 2-éthyl-1-butène dans un flux de produit alpha-oléfine linéaire, comprenant :

l'acheminement d'un flux de produit alpha-oléfine linéaire comprenant au moins une alpha-oléfine linéaire, telle que le 1-hexène et le 2-éthyl-1-butène, à un réacteur logeant un catalyseur à alumine modifié pour isomériser au moins une partie du 2-éthyl-1-butène en cis- ou trans-3-méthyl-2-pentène, la période pendant laquelle ledit acheminement se produit étant le temps en présence de flux ;
le retrait d'un effluent du réacteur contenant moins de 2-éthyl-1-butène que le flux de produit alpha-oléfine linéaire ; et

la régénération périodique du catalyseur à alumine modifié en arrêtant l'étape d'acheminement et en introduisant un environnement non oxydant dans le réacteur à des intervalles de temps en présence de flux n'excédant pas environ 200 heures, dans lequel le réacteur est soumis à l'environnement non oxydant pendant une période d'environ 15 heures ou plus et dans lequel l'environnement non oxydant a une température d'environ 250 °C ou plus pendant au moins une partie de la période de temps.

**2.** Procédé catalytique selon la revendication 1, dans lequel l'environnement non oxydant comprend un environnement sous vide ou un environnement de gaz inerte, tel que de l'azote ou un environnement de gaz noble.

**3.** Procédé catalytique selon la revendication 1 ou 2, dans lequel la régénération comprend le passage d'un gaz inerte à travers le réacteur en deux étapes séquentielles comprenant i) une première étape de température inférieure comprenant le passage du gaz inerte à travers le réacteur à une température de gaz inerte d'environ 200 °C ou moins pendant une période d'environ 2 heures ou plus ; et ii) une deuxième étape de température supérieure comprenant le passage du gaz inerte à travers le réacteur à une température de gaz inerte d'environ 250 °C ou plus pendant une période d'environ 8 heures ou plus.

**4.** Procédé catalytique selon la revendication 3, dans lequel la première étape de température inférieure comprend un ou plusieurs parmi :

le passage du gaz inerte à travers le réacteur à une température de gaz inerte d'environ 150 °C ou moins ;
le passage du gaz inerte à travers le réacteur à une température de gaz inerte d'environ 40 °C à environ 150 °C ;
le passage du gaz inerte à travers le réacteur à des températures de gaz inerte multiples allant d'environ 40 °C à environ 150 °C, dans lequel la température de gaz inerte est augmentée à chacune des températures de gaz inerte multiples à une vitesse d'environ 1 à environ 5 °C/min ;
le passage du gaz inerte à travers le réacteur pendant une période d'environ 4 heures ou plus ; et
le passage du gaz inerte à travers le réacteur pendant une période d'environ 2 à environ 6 heures.

**5.** Procédé catalytique selon la revendication 3, dans lequel la deuxième étape de température supérieure comprend un ou plusieurs parmi :

le passage du gaz inerte à travers le réacteur à une température de gaz inerte d'environ 300 °C ou moins ;
le passage du gaz inerte à travers le réacteur à une température de gaz inerte d'environ 250 °C à environ 300 °C ;
le passage du gaz inerte à travers le réacteur pendant une période d'environ 14 heures ou moins ; et
le passage du gaz inerte à travers le réacteur pendant une période d'environ 8 à environ 12 heures.

**6.** Procédé catalytique selon l'une quelconque des revendications 3 à 5, dans lequel le passage de la première étape de température inférieure à la deuxième étape de température supérieure se produit à une vitesse d'environ 1 à environ 5 °C/min.

**7.** Procédé catalytique selon l'une quelconque des revendications 3 à 6, dans lequel le débit de gaz inerte est d'environ 120 à environ 300 $cm^3$/min.

**8.** Procédé catalytique selon l'une quelconque des revendications 1 à 7, comprenant en outre la régénération périodique du catalyseur à alumine modifié en arrêtant l'étape d'acheminement et en introduisant un gaz oxydant dans le réacteur.

**9.** Procédé catalytique selon la revendication 8, dans lequel le gaz oxydant comprend de l'oxygène à une température de gaz d'environ 300 °C à environ 450 °C, introduisant facultativement le gaz oxydant pendant une période de 1 à 24 heures.

**10.** Procédé catalytique selon l'une quelconque des revendications 1 à 9, dans lequel le catalyseur à alumine modifié est capable de convertir environ 80 % en poids ou plus de 2-éthyl-1-butène en cis- ou trans-3-méthyl-2-pentène à une pression d'environ 0,1 - 10 barg, une température d'environ 40 - 100 °C, et une vitesse spatiale horaire liquide d'environ 0,5 - 10 $h^{-1}$, et dans lequel environ 3 % en poids ou moins de 1-hexène est converti en un isomère différent dans les mêmes conditions de réaction.

**11.** Procédé catalytique selon l'une quelconque des revendications 1 à 10, comprenant en outre l'acheminement de l'effluent du réacteur dans une colonne de distillation pour produire un flux aérien comprenant l'alpha-oléfine linéaire

et un flux de fond comprenant du cis- ou trans-3-méthyl-2-pentène.

**12.** Procédé catalytique selon la revendication 11, dans lequel le flux aérien comprend environ 99,5 % en poids de 1-hexène ou plus et environ 0,15 % en poids de 2-éthyl-1-butène ou un isomère de celui-ci ou moins.

**13.** Procédé catalytique selon l'une quelconque des revendications 1 à 12, dans lequel le catalyseur à alumine modifié présente environ 0,01 à environ 0,5 mmol/g de catalyseur de sites acides Brønsted tels que déterminés par spectroscopie FTIR à l'aide de pyridine comme molécule sonde.

**14.** Procédé catalytique selon la revendication 13, dans lequel le catalyseur à alumine modifié présente environ 0,04 à environ 0,2 mmol/g de catalyseur de sites acides Brønsted tels que déterminés par spectroscopie FTIR en utilisant la pyridine comme molécule sonde.

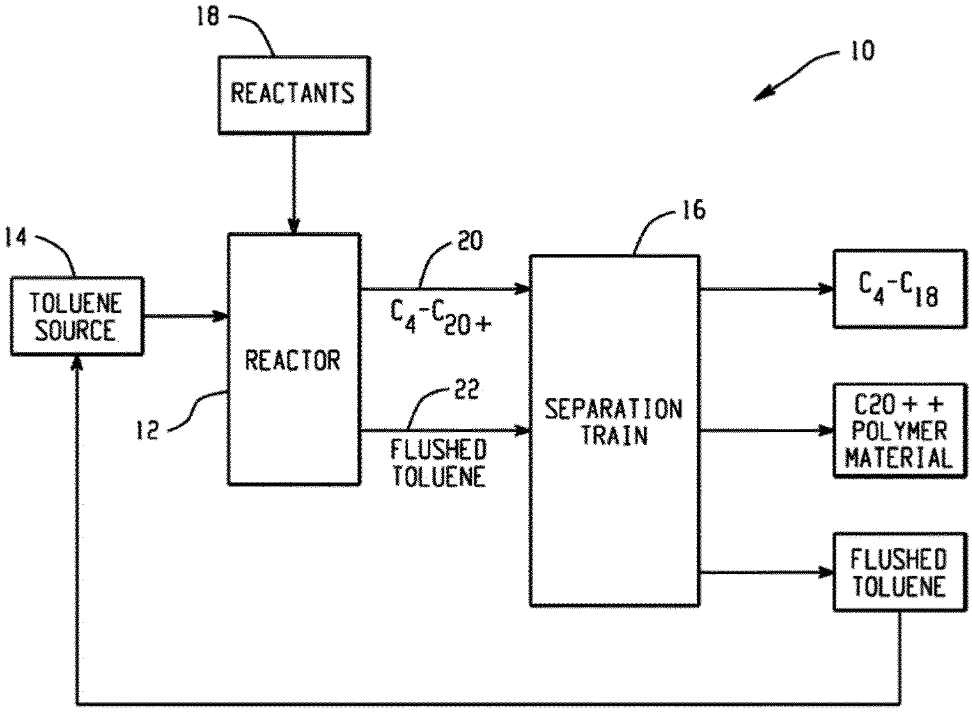
18
REACTANTS
10
14
TOLUENE SOURCE
REACTOR
12
20
$C_4$-$C_{20}$+
22
FLUSHED TOLUENE
16
SEPARATION TRAIN
$C_4$-$C_{18}$
C20++ POLYMER MATERIAL
FLUSHED TOLUENE

FIG. 1

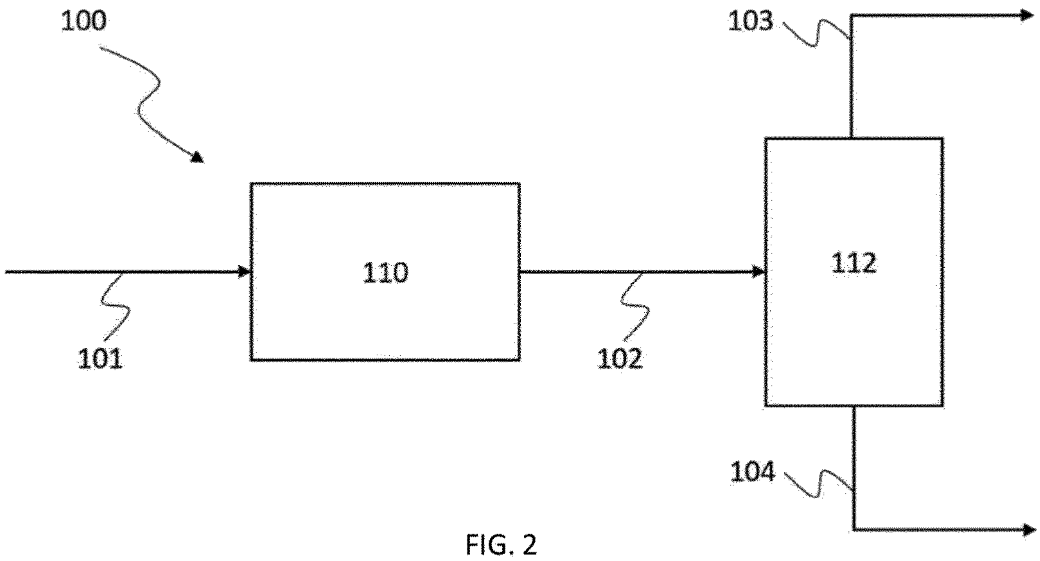


FIG. 2

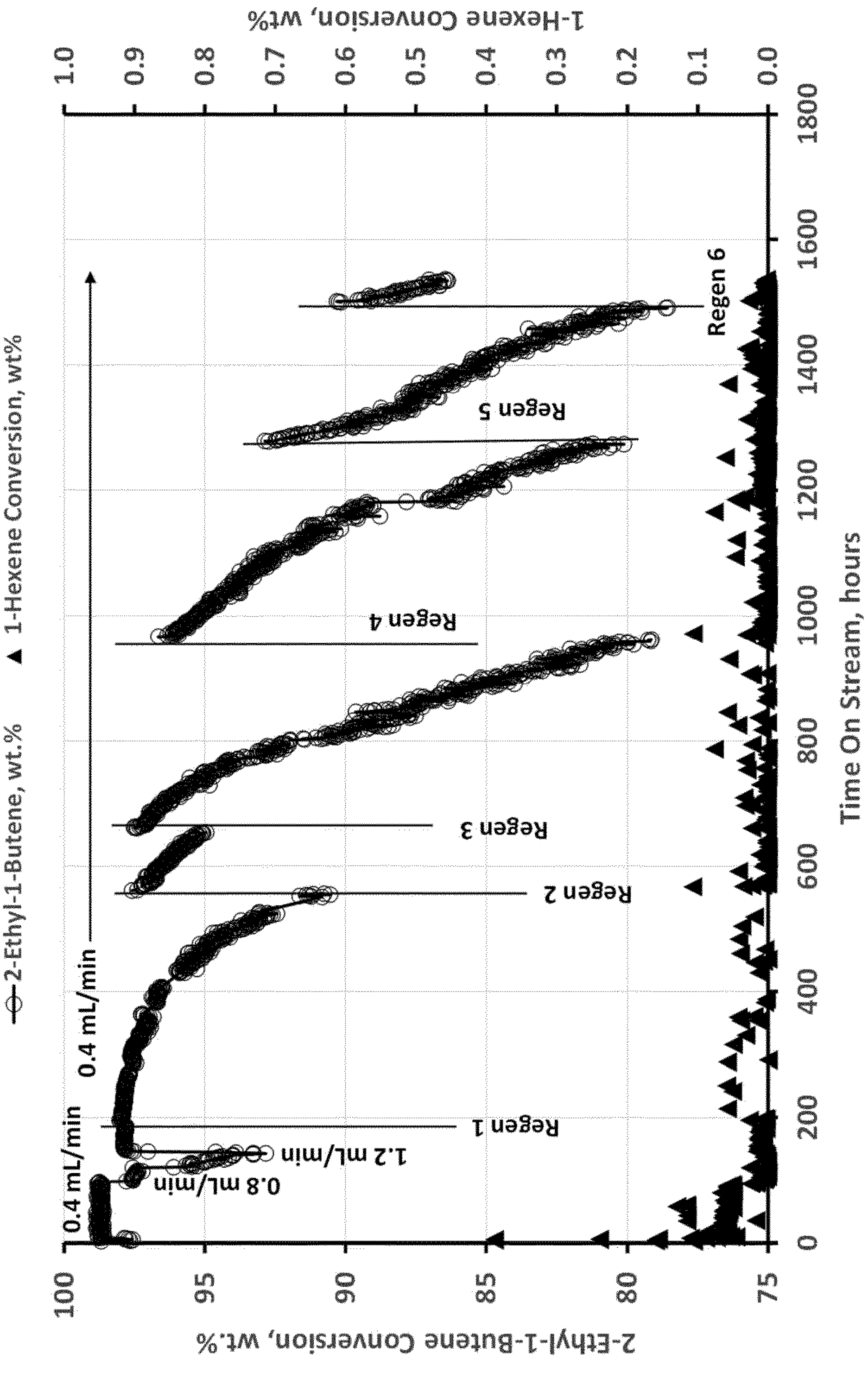
2-Ethyl-1-Butene, wt.%
1-Hexene Conversion, wt%
2-Ethyl-1-Butene Conversion, wt.%
Time On Stream, hours
0.4 mL/min
0.8 mL/min
1.2 mL/min
0.4 mL/min
Regen 1
Regen 2
Regen 3
Regen 4
Regen 5
Regen 6
100
95
90
85
80
75
0
200
400
600
800
1000
1200
1400
1600
1800
1.0
0.9
0.8
0.7
0.6
0.5
0.4
0.3
0.2
0.1
0.0

FIG. 3

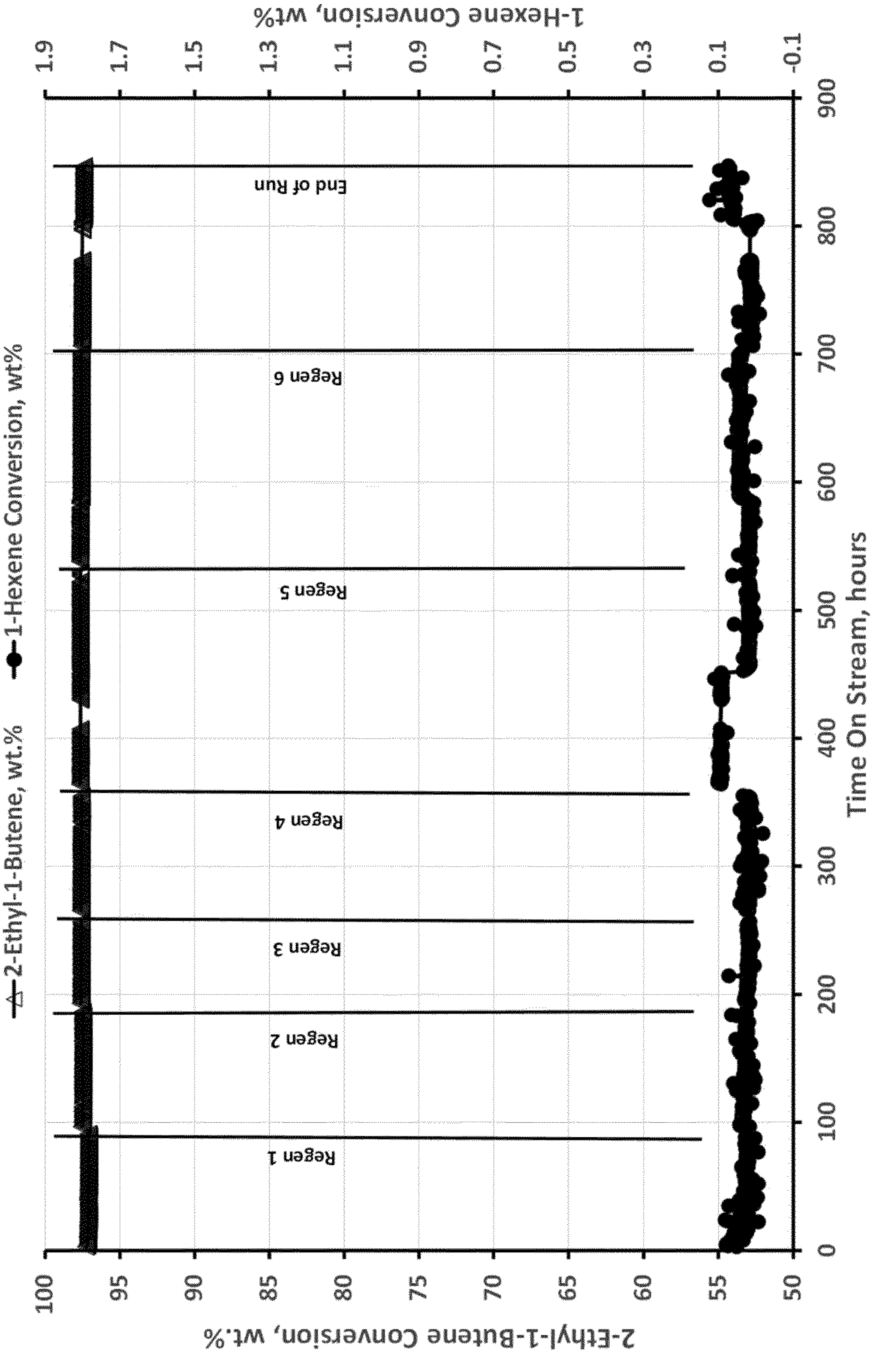
2-Ethyl-1-Butene, wt.%
1-Hexene Conversion, wt%
1-Hexene Conversion, wt%
2-Ethyl-1-Butene Conversion, wt.%
Time On Stream, hours
Regen 1
Regen 2
Regen 3
Regen 4
Regen 5
Regen 6
End of Run
100
95
90
85
80
75
70
65
60
55
50
1.9
1.7
1.5
1.3
1.1
0.9
0.7
0.5
0.3
0.1
-0.1
0
100
200
300
400
500
600
700
800
900

FIG. 4

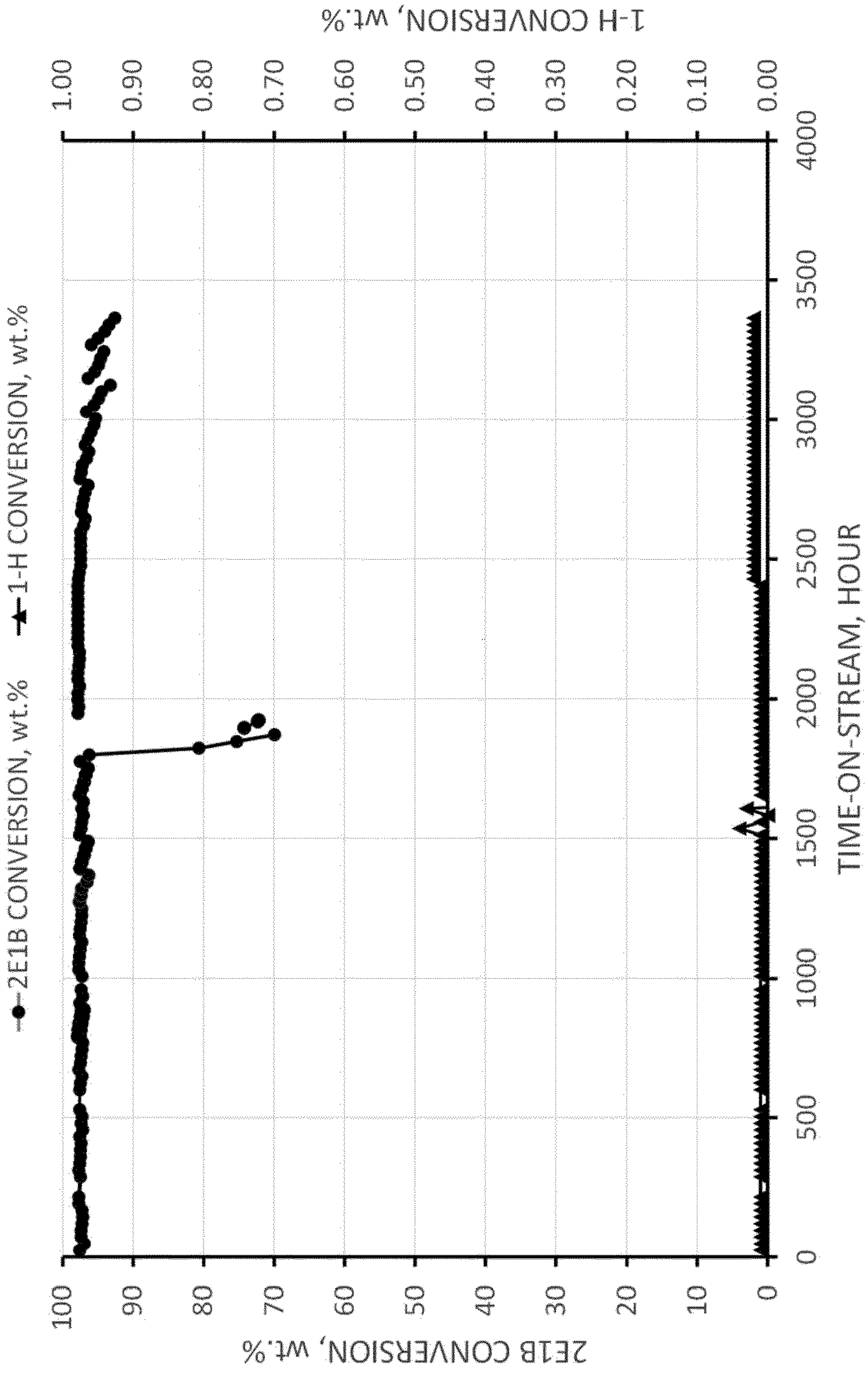
2E1B CONVERSION, wt.%
1-H CONVERSION, wt.%
1-H CONVERSION, wt.%
1.00
0.90
0.80
0.70
0.60
0.50
0.40
0.30
0.20
0.10
0.00
2E1B CONVERSION, wt.%
100
90
80
70
60
50
40
30
20
10
0
0
500
1000
1500
2000
2500
3000
3500
4000
TIME-ON-STREAM, HOUR

FIG. 5

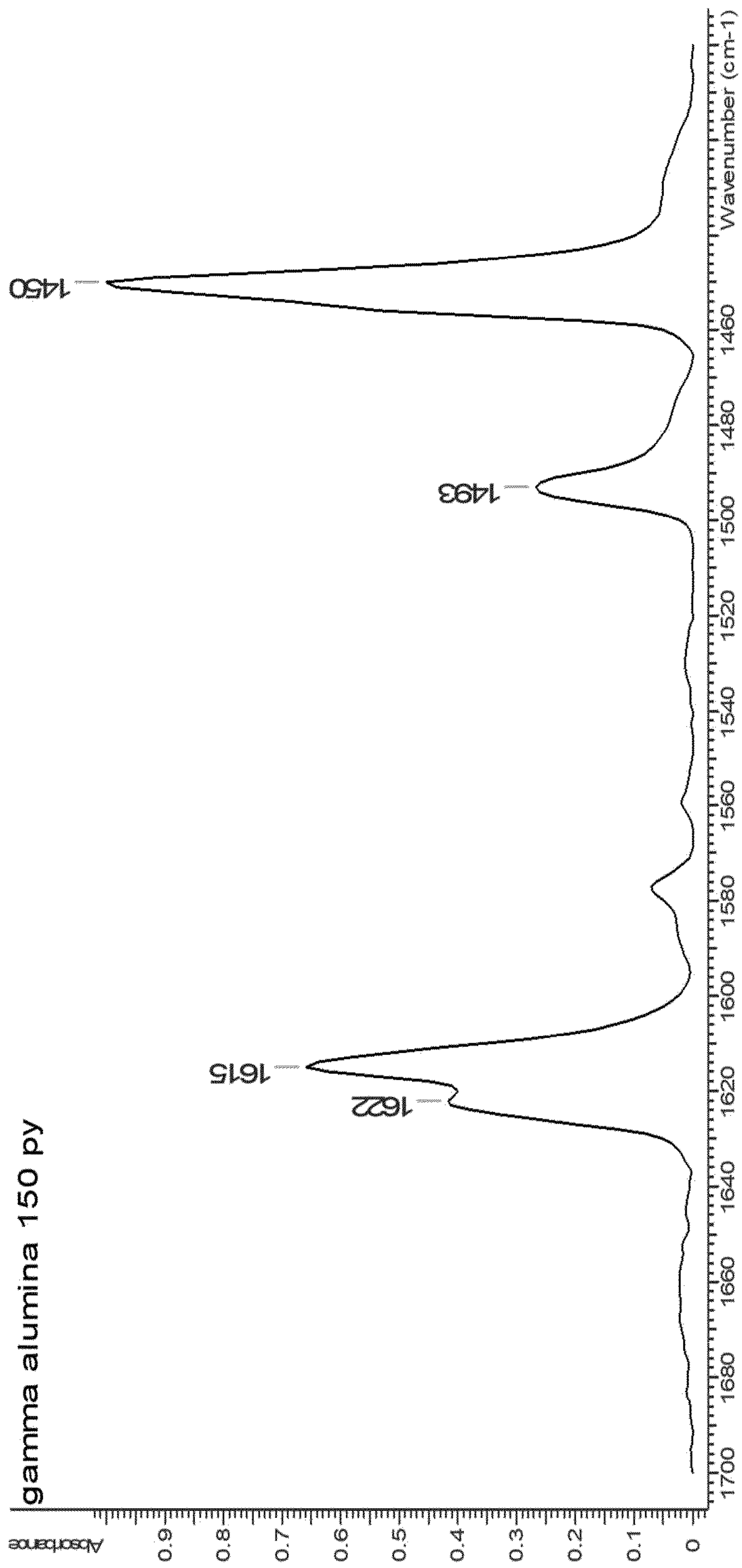
gamma alumina 150 py
Absorbance
0.9
0.8
0.7
0.6
0.5
0.4
0.3
0.2
0.1
0
1450
1493
1615
1622
1700
1680
1660
1640
1620
1600
1580
1560
1540
1520
1500
1480
1460
Wavenumber (cm-1)

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 2206557
- DE 10336015


### Non-patent literature cited in the description


- *J. Phys. Chem. C*, 2014, vol. 118 (12), 6226-6234
- **GABRIENKO, A.A. et al.** *Journal of Physical Chemistry*, 2018, vol. 122, 25386
- **EMEIS, C.A.** *Journal of Catalysis*, 1993, vol. 141, 347